# EUROPEAN PATENT APPLICATION

(11) **EP 4 471 127 A1**
(43) Date of publication of application: **04.12.2024**
(21) Application number: 23763126.2
(22) Date of filing: 13.01.2023
(51) Int. Cl.: C12N 5/0775, C12M 1/00, C12M 3/00

(54) **CULTURE DEVICE**

(30) Priority: 04.03.2022 JP 2022033494
(71) Applicant: PHC Corporation, Toon-shi Ehime 791-0395 (JP)
(72) Inventor: TAMEYORI, Yuuki, Toon-shi, Ehime 791-0395 (JP); OHTA, Akihiro, Toon-shi, Ehime 791-0395 (JP); AOKI, Hikaru, Toon-shi, Ehime 791-0395 (JP)
(74) Representative: Grünecker Patent- und Rechtsanwälte PartG mbB
(86) International application number: PCT/JP2023/000802
(87) International publication number: WO 2023/166863

(57) **Abstract**

This culture device comprises: a vaporizer that provides vapor to a culture chamber by vaporizing water that was dropped into the vaporizer; and a heater that heats the vaporizer. The vaporizer is provided with a water-droplet passage blocking mechanism, between an upstream region in which an inlet for the water and a bottom surface with which the water makes contact are present, and a downstream region in which an outlet for the vapor is present.

## Description

### Technical Field

The present disclosure relates to a culture apparatus.

### Background Art

In a culture apparatus (incubator) for incubating a culture such as a cell or a microorganism, it is necessary to cause the humidity in a culture chamber to be suitable for the incubation. For example, as indicated in Patent Literature (hereinafter referred to as "PTL") 1, humidification is performed using a humidification tray placed in a culture chamber in the related art. Water in the humidification tray vaporizes until the temperature in the culture chamber reaches the saturation humidity, and thus, the culture chamber is humidified.

### Citation List

### Patent Literature

PTL 1
Japanese Patent Application Laid-Open No. 2017-201886

### Summary of Invention

### Technical Problem

Water in the humidification tray gradually vaporizes. That is, in a case where the humidification tray is used, it is not possible to quickly humidify the culture chamber. Assuming that water in the humidification tray is boiled to promote vaporization of the water, there is a high possibility that water droplet adhesion due to condensation will occur in the culture chamber. The water droplet adhesion may cause contamination, such as the proliferation of miscellaneous germs.

An object of the present disclosure is to quickly humidify a culture chamber while preventing water droplet adhesion from occurring in the culture chamber.

### Solution to Problem

An aspect of a culture apparatus according to the present disclosure includes: a vaporizer that vaporizes water, which is dropped into the vaporizer, to supply vapor to a culture chamber; and a heater that heats the vaporizer. The vaporizer includes a water-droplet passage prevention mechanism between an upstream region, in which an inlet for the water and a bottom surface with which the water comes into contact are present, and a downstream region, in which an outlet for the vapor is present.

### Advantageous Effects of Invention

According to the present disclosure, it is possible to quickly humidify a culture chamber while preventing water droplet adhesion from occurring in a culture chamber.

### Brief Description of Drawings

FIG. 1 is a schematic longitudinal sectional view of a culture apparatus in an embodiment of the present disclosure as viewed from the right side;
FIG. 2 is a longitudinal sectional view of a main part of a vapor supply apparatus;
FIG. 3 is a cross-sectional arrow view taken along line III-III of FIG. 2;
FIG. 4 is a partial enlarged view of a vaporizer; and
FIG. 5 is a longitudinal sectional view of the vapor supply apparatus according to a variation.

### Description of Embodiments

Hereinafter, a culture apparatus according to an embodiment of the present disclosure will be described with reference to the accompanying drawings. The embodiment described below is merely an example, and is not intended to exclude the application of various modifications and technologies that are not explicitly described in the embodiment described below. In addition, each configuration of the embodiment can be variously modified and implemented without departing from the spirit thereof. Further, each configuration of the embodiment can be selected as necessary or can be combined as appropriate.

In the present specification, the front, rear, left, right, up and down of the culture apparatus are defined as follows. That is, the side of the culture apparatus, which the user faces when using the culture apparatus (the side on which outer door 3a and inner door 3b which are described later are present), will be referred to as the front of the culture apparatus, and the side opposite to the front will be referred to as the rear of the culture apparatus. In addition, the left and right of the culture apparatus are defined based on a case where the culture apparatus is viewed from the front to the rear. Further, the side of the culture apparatus, which is closer to the plane on which the culture apparatus is installed, will be referred to as the down of the culture apparatus, and the side opposite to the down will be referred to as the up of the culture apparatus.

Note that, in all the drawings provided for describing the embodiment, the same elements are in principle denoted by the same reference signs, and the description thereof may be omitted.

### [1. Overall Configuration]

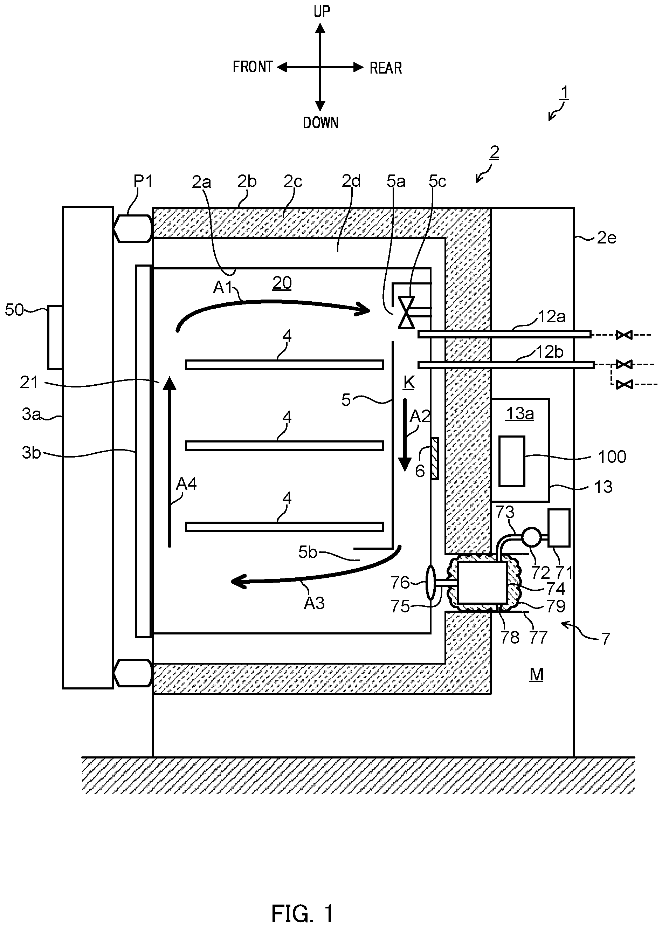

A culture apparatus in its entirety in an embodiment of the present invention will be described with reference to FIG. 1. FIG. 1 is a schematic longitudinal sectional view of a culture apparatus in an embodiment of the present invention as viewed from the right side.

Culture apparatus 1 illustrated in FIG. 1 is an apparatus for incubating a culture such as a cell or a microorganism. Culture apparatus 1 described above includes: housing 2 which has a substantially box shape and includes culture chamber 20 formed therein and opening 21 at the front surface thereof; and outer door 3a and inner door 3b both of which are for opening and closing opening 21. Culture chamber 20 is vertically partitioned by a plurality of shelves 4. Packing P1 is provided at an outer edge of outer door 3a.

In culture chamber 20, the temperature, the humidity, the O₂ (oxygen) concentration, and the CO₂ (carbon dioxide) concentration are maintained in appropriate ranges, respectively, such that culture chamber 20 has an appropriate atmosphere for incubating a culture.

Housing 2 includes: inner box 2a which has a substantially box shape and includes culture chamber 20 formed therein; and outer box 2b which has a substantially box shape and covers the outer side of inner box 2a.

Inner box 2a and outer box 2b are formed of a metal plate. Heat insulation material 2c is disposed between inner box 2a and outer box 2b. Heat insulation material 2c is formed, for example, by combining plate-shaped heat insulation materials. Heat insulation space 2d is formed between inner box 2a and heat insulation material 2c.

The rear surface and bottom surface of outer box 2b of housing 2 are covered with cover 2e. The space between the rear surface of outer box 2b and cover 2e constitutes mechanical room M for disposing various equipment therein. Mechanical room M is provided with electrical box 13. Control apparatus 100 and other electrical components (not illustrated) are housed in interior 13a of electrical box 13.

In culture chamber 20, duct 5 that vertically extends is disposed. Duct 5 is attachably/detachably attached to the rear surface of inner box 2a. Gas passage K is formed inside duct 5. In gas passage K described above, circulation fan 5c is disposed. By operating circulation fan 5c, the air in culture chamber 20 is sucked in through suction port 5a formed in an upper portion of duct 5, and this air is blown out to culture chamber 20 through blow-out port 5b provided in a lower portion of duct 5. Thus, forced circulation of the air as indicated with arrows A1, A2, A3, and A4 is performed.

In duct 5, gas supply apparatuses 12a and 12b for supplying culture chamber 20 with an adjustment gas (O₂ gas, N₂ gas, and CO₂ gas) for adjusting the O₂ gas concentration and the CO₂ gas concentration in culture chamber 20 are installed. Further, an environmental sensor that measures the temperature, the humidity, the O₂ gas concentration, and the CO₂ gas concentration in culture chamber 20 is installed in duct 5. Further, an ultraviolet irradiation apparatus that sterilizes the air in culture chamber 20 by ultraviolet irradiation may be installed in duct 5.

Culture chamber heaters 6 for temperature adjustment, that is, for controlling the temperature in culture chamber 20 are installed at the respective rear surfaces (the surfaces on the side of outer box 2b) of the right side wall, the left side wall, the rear wall, the top wall, and the bottom wall of inner box 2a. FIG. 1 illustrates culture chamber heater 6 installed at the rear wall as a representative example. Note that, culture chamber heater 6 is, in principle, in a state of being energized and generating heat while culture apparatus 1 is in operation. The output (heating force) of culture chamber heater 6 is controlled by control apparatus 100.

In inner box 2a, outer box 2b, and heat insulation material 2c, through-holes are formed, and vapor supply apparatus 7 is disposed so as to span over mechanical room M and the through-holes described above. Vapor supply apparatus 7 is an apparatus that supplies vapor to culture chamber 20 and humidifies culture chamber 20.

Further, culture apparatus 1 receives instructions to start and stop culture apparatus 1, operation mode settings, and inputs of various setting values for culture chamber 20 from operation apparatus 50 provided in outer door 3a. The various setting values for culture chamber 20 are a set temperature, a set humidity, a set O₂ gas concentration, a set CO₂ gas concentration, and the like. Control apparatus 100 controls the constituent elements such as circulation fan 5c, gas supply apparatuses 12a and 12b, culture chamber heaters 6, vapor supply apparatus 7, and the like based on the inputs from operation apparatus 50 and the environmental sensor. Operation apparatus 50 includes a display that displays the state of culture apparatus 1.

### [2. Vapor Supply Apparatus]

Vapor supply apparatus 7 includes tank 71, pump 72, supply pipe 73, vaporizer 74, discharge pipe 75, and nozzle member 76. Case 77 is fixed to the surface of outer box 2b on the side of mechanical room M. Support 78 that supports vaporizer 74 is attached to case 77. Vaporizer 74 is covered on the whole with heat-insulating coated product 79.

Tank 71 is a container for storing water. FIG. 1 illustrates a state in which tank 71 is disposed inside mechanical room M, but tank 71 may be installed outside culture apparatus 1 or may be disposed in a space that differs from mechanical room M included in culture apparatus 1. Pump 72 is a pump capable of delivering water in tank 71 little by little, and is, for example, a tube pump. Supply pipe 73 is a flexible tube formed of, for example, resin. An end of supply pipe 73, that is, the portion of supply pipe 73, which is connected to vaporizer 74, may be formed of metal. Pump 72 operates, and thus, the water in tank 71 is supplied to vaporizer 74 through supply pipe 73 little by little.

The water supplied to vaporizer 74 vaporizes inside vaporizer 74 to turn into vapor. The generated vapor passes through discharge pipe 75 and is supplied from nozzle member 76 to culture chamber 20.

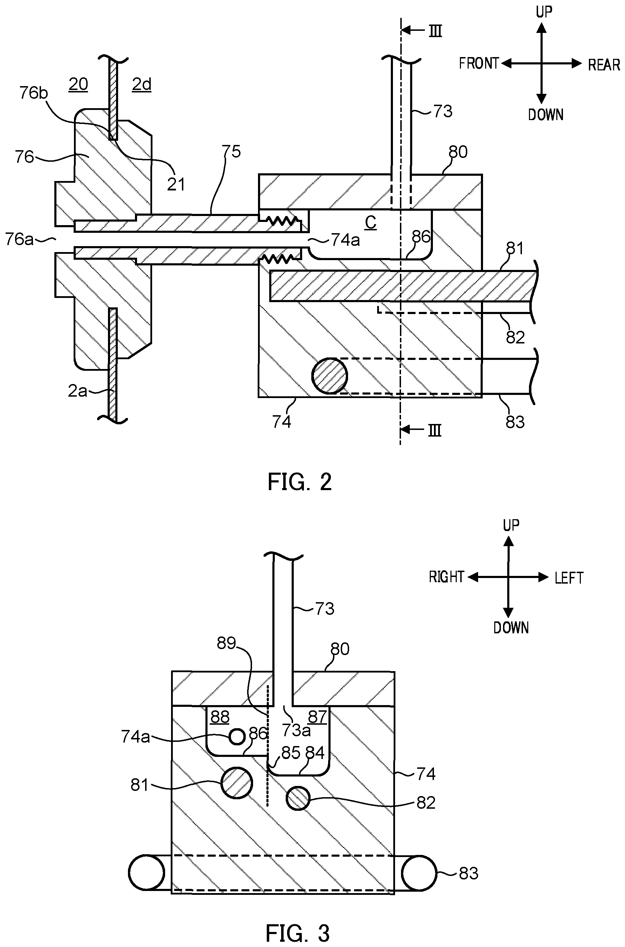

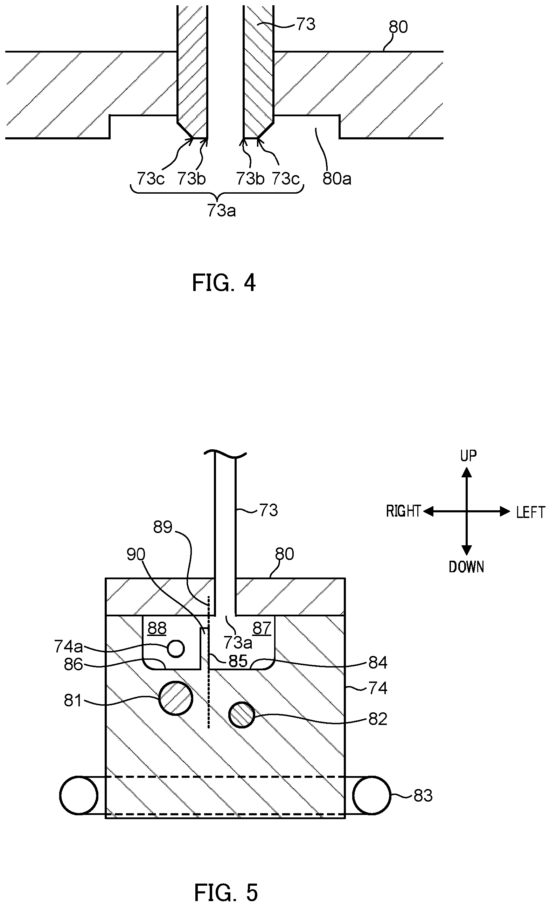

FIG. 2 is a longitudinal sectional view of a main part of vapor supply apparatus 7. FIG. 3 is a cross-sectional arrow view taken along line III-III of FIG. 2. FIG. 4 is a partial enlarged view of vaporizer 74.

Vaporizer 74 is a block-shaped member with a hollowed-out portion as vaporization chamber C. Vaporizer 74 is formed of a highly corrosion-resistant metal such as stainless steel. Vaporization chamber C opens on the upper side. Lid 80 is fixed to the upper side of vaporizer 74 so as to close the opening of vaporization chamber C. Lid 80 includes recess 80a formed in a portion to which supply pipe 73 is connected. Supply pipe 73 includes inlet 73a that is an opening connected to vaporization chamber C. Water is dropped into vaporization chamber C through inlet 73a.

A plurality of holes is formed in vaporizer 74, and heater 81, temperature sensor 82, and safety apparatus 83 are inserted into these holes. Heater 81 is a rod-like heater, for example, a ceramic heater, that heats vaporizer 74. Temperature sensor 82 detects the temperature of vaporizer 74 and transmits the detection result to control apparatus 100. Control apparatus 100 controls the heat value of heater 81 based on the detection result of temperature sensor 82. Safety apparatus 83 is, for example, a temperature fuse, and forcibly cuts off the power supply to heater 81 in a case where heater 81 overheats due to a failure of heater 81 or temperature sensor 82 and the temperature of vaporizer 74 becomes abnormally high.

Vaporization chamber C includes: bottom surface 84 that is located immediately below inlet 73a; wall surface 85 that rises from bottom surface 84; and second bottom surface 86 that continues from an upper end of wall surface 85. Outlet 74a that is an opening connected to discharge pipe 75 is formed at a side wall of vaporization chamber C, specifically, the side wall thereof, which is located on a side opposite to inlet 73a and bottom surface 84 across wall surface 85. Vapor generated in vaporization chamber C goes out to the outside of vaporization chamber C through outlet 74a.

Vaporization chamber C can be divided into upstream region 87, which is located on the upstream side of the flow of vapor, and downstream region 88, which is located on the downstream side of the flow of vapor. Wall surface 85 is present between upstream region 87 and downstream region 88. FIG. 3 illustrates virtual plane 89, which separates upstream region 87 and downstream region 88, with a dotted line. Wall surface 85 constitutes a part of virtual plane 89.

Upstream region 87 is a region in which inlet 73a, which is an opening through which water to be dropped enters, and bottom surface 84, which is a surface with which the dropped water comes into contact and in which the dropped water vaporizes, are present. In other words, upstream region 87 is a region in which vapor is actually generated. Downstream region 88 is a region in which outlet 74a, which is an opening through which the generated vapor goes out, is present. The shape of virtual plane 89 that separates upstream region 87 and downstream region 88 is not necessarily limited to that illustrated in FIG. 3.

Discharge pipe 75 is connected to vaporizer 74 so as to protrude from the outer surface of vaporizer 74 toward culture chamber 20. Discharge pipe 75 is formed of a highly corrosion-resistant metal such as stainless steel. The discharge pipe includes a passage that is connected to outlet 74a. The passage of the discharge pipe has a diameter equal to the diameter of outlet 74a.

Nozzle member 76 is attached to a leading end of discharge pipe 75. Nozzle member 76 is fixed to inner box 2a so as to be fitted into through-hole 21 formed at inner box 2a. In the case of the present embodiment, nozzle member 76 is fixed to inner box 2a via a component made of rubber (not illustrated). Specifically, nozzle member 76 includes locker 76b (specifically, a locking groove) having an annular shape on the outer peripheral surface. Locker 76b is fitted into through-hole 21 via the component made of rubber described above. The component made of rubber has, for example, an annular shape, and is provided between locker 76b and through-hole 21 over the entire circumference. Vapor discharging port 76a that is an opening through which vapor is discharged toward culture chamber 20 is formed in nozzle member 76. Vapor discharging port 76a constitutes a downstream end of the vapor passage connected from outlet 74a. Vapor discharging port 76a has a diameter larger than the diameter of outlet 74a.

Nozzle member 76 covers the leading end of discharge pipe 75 and is formed of a material, for example, a resin such as polycarbonate, which has a lower thermal conductivity than metal. Further, the component made of rubber provided between locker 76b of nozzle member 76 and through-hole 21 of inner box 2a is constituted by, for example, a rubber material (for example, silicone rubber) having a thermal conductivity equivalent to or lower than that of the resin that constitutes nozzle member 76. The configuration in which such a component made of rubber is provided makes it possible to suppress heat transfer from discharge pipe 75 to inner box 2a via nozzle member 76, to enhance the airtightness of the fitting portion of nozzle member 76, and further to suppress gas leakage in inner box 2a. Note that, the compartment made of rubber described above may be omitted. That is, nozzle member 76 may be directly fixed to inner box 2a.

Vapor supply apparatus 7 configured as described above operates as follows. When it is necessary to humidify culture chamber 20, control apparatus 100 operates pump 72. Control apparatus 100 is configured to be capable of adjusting the speed of water delivery by pump 72. When pump 72 operates, the water in tank 71 is supplied into vaporization chamber C through inlet 73a.

Further, inlet 73a is constituted by inner edge 73b, through which water passes, and outer edge 73c, and recess 80a having a recessed shape is formed between outer edge 73c and lid 80. Accordingly, the surface tension of water supplied through inlet 73a is kept constant by the circumferential length of outer edge 73c. Thus, it is possible to keep the size of water droplet constant. Note that, in the present embodiment, outer edge 73c has a circular shape with a diameter of 2 mm, and inlet 73a makes it possible to drop water droplets of approximately 0.03 g per droplet. By supplying the water in tank 71 one drop at a time into vaporization chamber C through inlet 73a by pump 72, it is possible to generate a constant amount of vapor at all times. Thus, it is possible to suppress the supply of excessive vapor to culture chamber 20 and to suppress water droplet adhesion in culture chamber 20 due to condensation.

Water supplied into vaporization chamber C through inlet 73a becomes water droplets and drops onto bottom surface 84 one drop at a time. Bottom surface 84 is heated by heater 81. Thus, when water droplets come into contact with bottom surface 84, the water droplets quickly vaporize and vapor is generated. The vapor generation causes the pressure in vaporization chamber C to rise momentarily, which is higher than the pressure in culture chamber 20. Thus, the vapor leaves vaporization chamber C through outlet 74a, passes through discharge pipe 75, and is discharged into culture chamber 20 through vapor discharging port 76a.

Control apparatus 100 is capable of adjusting the speed of change in the humidity in culture chamber 20 by adjusting the speed of water delivery (that is, the dropping number per unit time) by pump 72. Thus, by increasing the speed of water delivery by pump 72, it is possible to increase the humidity in culture chamber 20 more quickly. Further, since vapor is generated in vaporization chamber C different from culture chamber 20 and only the generated vapor is supplied to culture chamber 20, water droplet adhesion is not generated in the culture chamber.

Note that, control apparatus 100 may operate pump 72 provided that a door, specifically outer door 3a or inner door 3b, is closed. This configuration makes it possible to prevent high-temperature vapor from being discharged into culture chamber 20 while the door is open and the user is working in culture chamber 20. Control apparatus 100 can determine that the door is closed, based on the detection result of a door sensor, an operation on operation apparatus 50, and/or the like.

When water droplets fall and come into contact with bottom surface 84, the Leidenfrost phenomenon may occur. When the Leidenfrost phenomenon occurs, the water droplets that have come into contact with bottom surface 84 become spherical and irregularly move in vaporization chamber C. At this time, the spherical water droplets may burst, enter outlet 74a, and reach culture chamber 20 as they are. When the spherical water droplets reach culture chamber 20, it may result in a state in which the water droplets adhere to the inner surface of inner box 2a or the surface of duct 5, which may cause contamination.

Vaporizer 74, however, includes, at a boundary between upstream region 87 and downstream region 88, wall surface 85 that rises from bottom surface 84. Accordingly, assuming that the Leidenfrost phenomenon occurs, the spherical water droplets that move on bottom surface 84 are blocked by wall surface 85 and cannot move to downstream region 88. That is, it is possible to preliminarily prevent the spherical water droplets from bursting and entering outlet 74a, and further from reaching culture chamber 20 as they are, and to prevent water droplet adhesion from occurring in culture chamber 20. That is, wall surface 85 that rises from bottom surface 84 is an aspect of the water-droplet passage prevention mechanism that prevents water droplets from passing from upstream region 87 to downstream region 88.

Note that, in the present embodiment, heater 81 is disposed in a position which is at a height substantially identical to a height of bottom surface 84 and on a lateral side (the right-side in the case of the present embodiment) of bottom surface 84. In other words, heater 81 is disposed in a position that intersects with a horizontal plane including bottom surface 84. That is, the position in which heater 81 is disposed is a position close to bottom surface 84. Thus, heat generated by heater 81 can be efficiently transferred to bottom surface 84.

In addition, heater 81 has a rod-like shape. Heater 81 is disposed such that a leading end (specifically, the front end) of heater 81 is located closer to culture chamber 20 than outlet 74a. Thus, the passage, through which vapor flows, between outlet 74a and vapor discharging port 76a can be heated with a portion on the side of the leading end of heater 81. Accordingly, it is possible to prevent vapor from cooling and condensing in the passage after leaving vaporization chamber C. Thus, it is possible to preliminarily prevent water droplets generated by condensation from entering culture chamber 20 together with a flow of vapor generated in vaporization chamber C. Note that, heater 81 may be disposed so as to penetrate vaporizer 74.

In the case of the present embodiment, second bottom surface 86 is located in a position higher than that of bottom surface 84. Thus, by disposing heater 81 in a position immediately below second bottom surface 86, it is possible to bring the portion on the side of the leading end of heater 81 close to the passage, through which vapor flows, to heat the passage more efficiently. In addition, by bringing heater 81 close to second bottom surface 86, it is possible to cause the height of heater 81 to be substantially identical to the height of bottom surface 84 to bring heater 81 close to bottom surface 84. Accordingly, it is possible to efficiently heat bottom surface 84, with which water droplets come into contact and in which the water droplets vaporize, with heater 81 while efficiently heating the passage, through which vapor flows, with heater 81.

In addition, in the present embodiment, temperature sensor 82 is disposed immediately below bottom surface 84, that is, close to bottom surface 84. Thus, temperature sensor 82 can detect the temperature of bottom surface 84 accurately. Further, control apparatus 100 can control heater 81 such that the heat value of heater 81 is appropriate. Note that, stainless steel has a relatively low thermal conductivity among metals. Accordingly, when the distance between bottom surface 84 and temperature sensor 82 increases, it is difficult to detect the temperature of bottom surface 84 accurately. In the present embodiment, however, temperature sensor 82 is disposed immediately below bottom surface 84, and thus, temperature sensor 82 can detect the temperature of bottom surface 84 accurately even when vaporizer 74 is formed of a material, such as stainless steel, which has a relatively low thermal conductivity.

Further, in the present embodiment, nozzle member 76 is formed of a material having a lower thermal conductivity than metal. Accordingly, at least the surface of nozzle member 76, which faces culture chamber 20, has a lower temperature than vaporizer 74 and discharge pipe 75. Thus, even when vaporizer 74 is heated by heater 81 and further discharge pipe 75 is heated, it is possible to prevent a hand of the user who is working in culture chamber 20 from coming into contact with a high-temperature portion and from burning. That is, nozzle member 76 has a function as a burn preventing member. In addition, nozzle member 76 can prevent a temperature distribution bias from occurring in culture chamber 20 by culture chamber 20 being locally heated by the heat from heater 81 through discharge pipe 75.

In addition, nozzle member 76 has a lower temperature than vaporizer 74 and discharge pipe 75. For this reason, when vapor comes into contact with nozzle member 76, condensation occurs on the surface of nozzle member 76. In the present embodiment, however, vapor discharging port 76a has a diameter larger than the diameter of outlet 74a. Thus, vapor that has passed through the passage and has reached vapor discharging port 76a is ejected into culture chamber 20 without coming into contact with nozzle member 76. Further, when the flow path of vapor expands, the pressure of the vapor decreases and condensation is less likely to occur, and thus, the vapor is less likely to become water droplets. Thus, it is possible to ensure that the occurrence of condensation on the surface of nozzle member 76 is prevented.

Note that, in the present embodiment, outlet 74a has a diameter of 6 mm, vapor discharging port 76a has a diameter of 3 mm, and outer edge 73c of inlet 73a has a diameter of 2 mm, and the diameter of vapor discharging port 76a is larger than the diameter of outlet 74a, and the diameter of outlet 74a is larger than the diameter of outer edge 73c of inlet 73a. Thus, the pressure in vaporization chamber C when water droplets vaporize does not become too high, and vapor is ejected into culture chamber 20 through outlet 74a and vapor discharging port 76a. Since condensation is more likely to occur as the pressure of vapor increases, it is possible to supply vapor to culture chamber 20 without generating water droplets.

Note that, since heater 81 is a heater for vaporizing water, the temperature of heater 81 is much higher than that of culture chamber heater 6 which causes the temperature in culture chamber 20 to be suitable for incubation. Accordingly, when the heat of heater 81 is transferred to culture chamber 20, a bias may occur in the temperature distribution in culture chamber 20. In the present embodiment, however, heater 81 is disposed away from inner box 2a. Specifically, heater 81 is disposed such that heat insulation space 2d is interposed between heater 81 and inner box 2a. Thus, it is possible to prevent a temperature distribution bias from occurring in culture chamber 20 by the heat generated by heater 81 being transferred to culture chamber 20. This effect can be enhanced by heat-insulating coated product 79 (see FIG. 1) which covers vaporizer 74.

### [3. Variation]

The culture apparatus according to the present disclosure is not limited to that in the embodiment described above, and various modifications can be made within the scope not departing from the spirit thereof.

For example, vaporizer 74 may have the structure illustrated in FIG. 5. FIG. 5 is a longitudinal sectional view corresponding to FIG. 3. Vaporizer 74 illustrated in FIG. 5 differs from vaporizer 74 in the embodiment described above in the following point. That is, wall surface 85 that rises from bottom surface 84 is formed as a side surface of screen 90 disposed in vaporization chamber C. A gap is formed between the upper end of screen 90 and lid 80. Further, second bottom surface 86 is formed in a position at a height identical to a height of bottom surface 84. In the variation illustrated in FIG. 5, it is also possible to prevent, even when the Leidenfrost phenomena occurs in upstream region 87, the spherical water droplets from moving from upstream region 87 to downstream region 88 while moving vapor generated in upstream region 87 to downstream region 88. Screen 90 including wall surface 85 is an aspect of the water-droplet passage prevention mechanism that prevents water droplets from passing from upstream region 87 to downstream region 88.

Further, the water-droplet passage prevention mechanism that prevents water droplets from passing from upstream region 87 to downstream region 88 may not necessarily include wall surface 85 that rises from bottom surface 84. For example, the water-droplet passage prevention mechanism may be constituted by a mesh member having a mesh size that makes it possible to capture water droplets while allowing vapor to passing through. Vaporizer 74 including wall surface 85 illustrated in FIG. 3 or 5 may further include such a mesh member.

Further, in the vaporization chamber, the upstream region may be located on the rear side, and the downstream region may be located on the front side. That is, wall surface 85 may be disposed so as to be orthogonal to the direction in which discharge pipe 75 extends (the front-rear direction).

The disclosure of Japanese Patent Application No. 2022-033494, filed on March 4, 2022, including the specification, drawings and abstract, is incorporated herein by reference in its entirety.

### Industrial Applicability

The present disclosure is suitably utilized as a culture apparatus for incubating a culture such as a cell or a microorganism.

### Reference Signs List

1 Culture apparatus
2 Housing
2a Inner box
21 Through-hole
2b Outer box
2c Heat insulation material
2d Heat insulation space
2e Cover
3 a Outer door
3b Inner door
4 Shelf
5 Duct
5a Suction port
5b Blow-out port
5c Circulation fan
6 Culture chamber heater
7 Vapor supply apparatus
12a, 12b Gas supply apparatus
13 Electrical box
20 Culture chamber
21 Opening
50 Operation apparatus
71 Tank
72 Pump
73 Supply pipe
73a Inlet
73b Inner edge
73c Outer edge
74 Vaporizer
74a Outlet
75 Discharge pipe
76 Nozzle member
76a Vapor discharging port
76b Locker
77 Case
78 Support
79 Heat-insulating coated product
80 Lid
80a Recess
81 Heater
82 Temperature sensor
83 Safety apparatus
84 Bottom surface
85 Wall surface
86 Second bottom surface
87 Upstream region
88 Downstream region
89 Virtual plane
90 Screen
100 Control apparatus
K Gas passage
P1 Packing
M Mechanical room
W Water
C Vaporization chamber

## Claims

1. A culture apparatus, comprising:
a vaporizer that vaporizes water to supply vapor to a culture chamber, the water being dropped into the vaporizer; and
a heater that heats the vaporizer, wherein
the vaporizer includes a water-droplet passage prevention mechanism between an upstream region and a downstream region, the upstream region being a region in which an inlet for the water and a bottom surface are present, the downstream region being a region in which an outlet for the vapor is present, the bottom surface being a surface with which the water comes into contact.

2. The culture apparatus according to claim 1, wherein
the water-droplet passage prevention mechanism includes a wall surface that rises from the bottom surface at a boundary between the upstream region and the downstream region.

3. The culture apparatus according to claim 2, wherein
the vaporizer includes, in the downstream region, a second bottom surface that continues from an upper end of the wall surface.

4. The culture apparatus according to claim 2, wherein
the water-droplet passage prevention mechanism includes a screen that includes the wall surface.

5. The culture apparatus according to any one of claims 1 to 4, wherein
the water-droplet passage prevention mechanism includes a mesh member disposed at a boundary between the upstream region and the downstream region.

6. The culture apparatus according to any one of claims 1 to 5, wherein
the heater is disposed in a position, the position being at a height substantially identical to a height of the bottom surface and on a lateral side of the bottom surface.

7. The culture apparatus according to any one of claims 1 to 6, wherein
the heater has a rod-like shape, and a leading end of the heater is located closer to the culture chamber than the outlet.

8. The culture apparatus according to any one of claims 1 to 7, further comprising:
an inner box that surrounds the culture chamber; and
a nozzle member that is fixed to the inner box and includes a vapor discharging port which is a downstream end of a vapor passage connected from the outlet.

9. The culture apparatus according to claim 8, wherein
the vapor discharging port has a diameter larger than a diameter of the outlet.

10. The culture apparatus according to claim 8 or 9, wherein
the heater is disposed away from the inner box.

11. The culture apparatus according to any one of claims 1 to 10, further comprising a temperature sensor disposed immediately below the bottom surface, wherein
the heater is configured such that a heat value of the heater changes based on a detection result of the temperature sensor.

12. The culture apparatus according to any one of claims 1 to 11, further comprising:
a door for opening and closing the culture chamber;
a pump that supplies the water to the inlet; and
a control apparatus that operates the pump provided that the door is closed.
